(19) Europäisches Patentamt
European Patent Office
Office européen des brevets

(11) Veröffentlichungsnummer: **0 098 953**
**B1**

(12) # EUROPÄISCHE PATENTSCHRIFT

(45) Veröffentlichungstag der Patentschrift: **13.08.86**

(51) Int. Cl.⁴: **C 07 D 207/456,** A 01 N 43/36

(21) Anmeldenummer: **83105351.7**

(22) Anmeldetag: **31.05.83**

(54) **Substituierte Maleinsäureimide, Verfahren zu ihrer Herstellung und ihre Verwendung als Schädlingsbekämpfungsmittel.**

(30) Priorität: **12.06.82 DE 3222152**

(43) Veröffentlichungstag der Anmeldung: **25.01.84 Patentblatt 84/4**

(45) Bekanntmachung des Hinweises auf die Patenterteilung: **13.08.86 Patentblatt 86/33**

(84) Benannte Vertragsstaaten: **AT BE CH DE FR GB IT LI NL SE**

(56) Entgegenhaltungen:
**EP-A-0 045 907**
**DE-A-1 695 306**
**GB-A-880 555**
**US-A-3 129 225**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Marzolph, Gerhard, Dr., Roggendorfstrasse 65, D-5000 Köln 80 (DE)**
Erfinder: **Blank, Heinz Ulrich, Dr., Am Geusfelde 35, D-5068 Odenthal (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen (DE)**
Erfinder: **Scheinpflug, Hans, Dr., Am Thelenhof 15, D-5090 Leverkusen (DE)**

**EP 0 098 953 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Substituierte Maleinsäureimide, Verfahren zu ihrer Herstellung und ihre Verwendung als Schädlingsbekämpfungsmittel

Die vorliegende Erfindung betrifft neue substituierte Maleinsäureimide, mehrere Verfahren zu ihrer Herstellung und ihre Verwendung als Schädlingsbekämpfungsmittel.

Es ist bereits bekannt, daß N-sulfenylierte Dicarbonsäureimide, wie z.B. N-Trichlormethylthiotetrahydrophthalimid, eine fungizide Wirksamkeit besitzen (vgl. US—A—2 553 770).

Weiterhin ist bekannt, daß N-Benzylmaleinsäureimide, z.B. N-[2'-Methylbenzyl]-3,4-dichlor-maleinsäureimid, fungizide Eigenschaften aufweisen (vgl. veröffentlichte Japan. Patent Anmeldung Nr. 52—93765).

Weiterhin sind N-Aralkyl-maleinsäureimide, wie N-[1-Methyl-2-phenyl-ethyl]-3,4-dichlormaleinsäureimid und N-(1,1-Diphenylmethyl)-3,4-dichlormaleinsäureimid bekannt (vgl. US—A—3 129 225). Über ihre Verwendung als Schädlingsbekämpfungsmittel ist nichts bekannt.

Es wurden neue substituierte Maleinsäureimide der Formel (I) gefunden

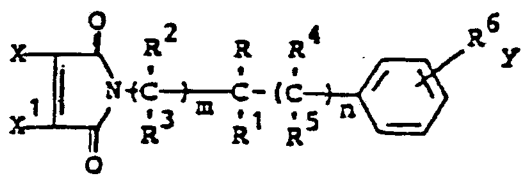

(I)

in welcher

X und $X^1$ für Chlor,

R für geradkettiges oder verzweigtes Alkyl mit 1 bis 4 Kohlenstoffatomen, wie Methyl, Ethyl, n-Propyl, iso-Propyl, n-Butyl, sec.-Butyl, iso-Butyl und tert.-Butyl, für gegebenenfalls ein- bis dreifach, gleich oder verschieden durch Methyl, Ethyl, Chlor und Brom substituiertes Phenyl, Benzyl bzw. Phenethyl,

$R^1$ bis $R^5$ gleich oder verschieden sind und für Wasserstoff, geradkettiges oder verzweigtes Alkyl mit 1 bis 4 Kohlenstoffatomen, wie Methyl, Ethyl, n-Propyl, iso-Propyl, n-Butyl, iso-Butyl, sec.-Butyl und tert.-Butyl, für gegebenenfalls ein- bis dreifach, gleich oder verschieden durch Methyl, Ethyl und Chlor substituiertes Phenyl, Benzyl bzw. Phenethyl,

$R^6$ für Chlor, Brom, Alkyl mit 1 bis 4 Kohlenstoffatomen, wie Methyl, Ethyl, n-Propyl, iso-Propyl, n-Butyl, sec.-Butyl, iso-Butyl und tert.-Butyl, für Alkoxy mit 1 bis 3 Kohlenstoffatomen, wie Methoxy, Ethoxy, n-Propoxy und iso-Propoxy, für Sulfonylamin, Alkylsulfonyl bzw. Alkylcarbonyl mit 1 bis 3 Kohlenstoffatomen im Alkylteil, wie Methylsulfonyl, Ethylsulfonyl, n-Propylsulfonyl, iso-Propylsulfonyl, Methylcarbonyl, Ethylcarbonyl, n-Propylcarbonyl und iso-Propylcarbonyl, für Carbonylamin, Trifluormethyl, Phenyl, Cyclopentyl oder Cyclohexyl,

m und n für 0, 1, 2 oder 3 stehen, die Summe aus

n und m für 0, 1 oder 2 und

y für 0, 1, 2 oder 3 stehen, ausgenommen die Verbindungen, in denen

X, $X^1$ für Chlor,

m und y für 0

n für 1

$R^1$, $R^4$ und $R^5$ für Wasserstoff und

R für Methyl und

X, $X^1$ für Chlor,

m, n und y für 0, $R^1$ für Wasserstoff und R für Phenyl stehen.

Die Verbindungen der Formel (I) können als Isomerengemisch oder in Form der einezelnen optischen Antipoden vorliegen.

Weiterhin wurde gefunden, daß man die substituierten Maleinsäureimide der Formel (I)

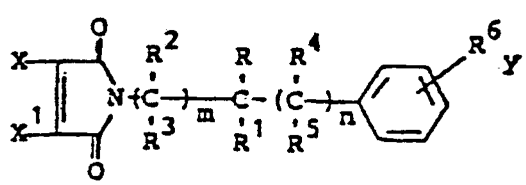

(I)

in welcher

X und $X^1$ fur Chlor,

R für geradkettiges oder verzweigtes Alkyl mit 1 bis 4 Kohlenstoffatomen, wie Methyl, Ethyl, n-Propyl,

iso-Propyl, n-Butyl, sec.-Butyl, iso-Butyl und tert.-Butyl, für gegebenenfalls ein- bis dreifach, gleich oder verschieden durch Methyl, Ethyl, Chlor und Brom substituiertes Phenyl, Benzyl bzw. Phenethyl,

$R^1$ bis $R^5$ gleich oder verschieden sind und für Wasserstoff, geradkettiges oder verzweigtes Alkyl mit 1 bis 4 Kohlenstoffatomen, wie Methyl, Ethyl, n-Propyl, iso-Propyl, n-Butyl, iso-Butyl, sec.-Butyl und tert.-Butyl, für gegebenenfalls ein- bis dreifach, gleich oder verschieden durch Methyl, Ethyl und Chlor substituiertes Phenyl, Benzyl bzw. Phenethyl,

$R^6$ für Chlor, Brom, Alkyl mit 1 bis 4 Kohlenstoffatomen, wie Methyl, Ethyl, n-Propyl, iso-Propyl, n-Butyl, sec.-Butyl, iso-Butyl und tert.-Butyl, für Alkoxy mit 1 bis 3 Kohlenstoffatomen, wie Methoxy, Ethoxy, n-Propoxy und iso-Propoxy, für Sulfonylamin, Alkylsulfonyl bzw. Alkylcarbonyl mit 1 bis 3 Kohlenstoffatomen im Alkylteil, wie Methylsulfonyl, Ethylsulfonyl, n-Propylsulfonyl, iso-Propylsulfonyl, Methylcarbonyl, Ethylcarbonyl, n-Propylcarbonyl und iso-Propylcarbonyl, für Carbonylamin, Trifluormethyl, Phenyl, Cyclopentyl oder Cyclohexyl,

m und n für 0, 1, 2 oder 3 stehen, die Summe aus
n und m für 0, 1 oder 2 und
y für 0, 1, 2 oder 3 stehen, ausgenommen die Verbindungen, in denen
X, $X^1$ für Chlor,
m und Y für O,
n für 1,
$R^1$, $R^4$ und $R^5$ für Wasserstoff und
R für Methyl stehen und
X, $X^1$ für Chlor,
m, n und y für 0 und
$R^1$ für Wasserstoff und
R für Phenyl stehen, erhält, wenn man
a) ein Halogenmaleinsäureanhydrid der Formel (II)

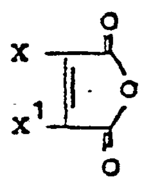

(II)

in welcher
X und $X^1$ die oben angegebene Bedeutung haben, mit primären Aminen der Formel (III)

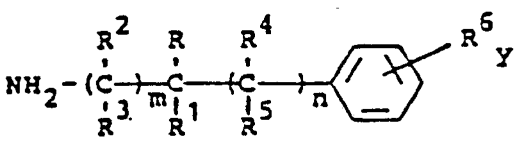

(III)

in welcher
R bis $R^6$, m, n und y die oben angegebene Bedeutung haben,
in einem Verdünnungsmittel umsetzt, oder
b) einen Halogenmaleinsäuredialkylester der Formel (IV)

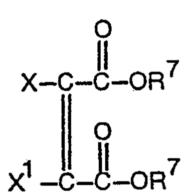

(IV)

in welcher
X und $X^1$ die oben angegebene Bedeutung haben, und
$R^7$ fur Alkyl steht,
mit primären Aminen der Formel (III), in welcher
R bis $R^6$, m, n und y die oben angegebene Bedeutung haben,
gegebenenfalls in einem Lösungs- oder Verdünnungsmittel umsetzt, oder
c) Halogenmaleinsäuremonoamide der Formel (V)

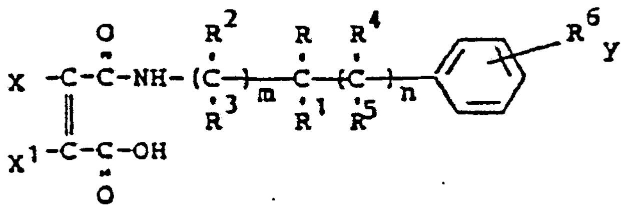

(V)

in welcher

X, $X^1$, R bis $R^6$, m, n und y die oben angegebene Bedeutung haben,

in Gegenwart eines Lösungsmittels, wie z.B. Eisessig und gegebenenfalls eines anhydridisierenden Mittels, wie z.B. Acetanhydrid oder Thionylchlorid zu den Verbindungen der Formel (I) cyclisiert.

Die neuen substituierten Maleinsäureimide weisen starke fungizide Eigenschaften auf. Überraschenderweise zeigen dabei die erfindungsgemäßen Verbindungen der Formel (I) eine erheblich höhere Wirkung als die aus dem Stand der Technik bekannten Verbindungen, welche wirkungsmäßig naheliegende Verbindungen sind.

Verwendet man beispielsweise für die Verfahrensvariante a) Dibrommaleinsäureanhydrid und 3-Phenyl-1,2,3,3-tetra-methylpropylamin, so kann der Reaktionsverlauf durch das folgende Formelschema wiedergegeben werden:

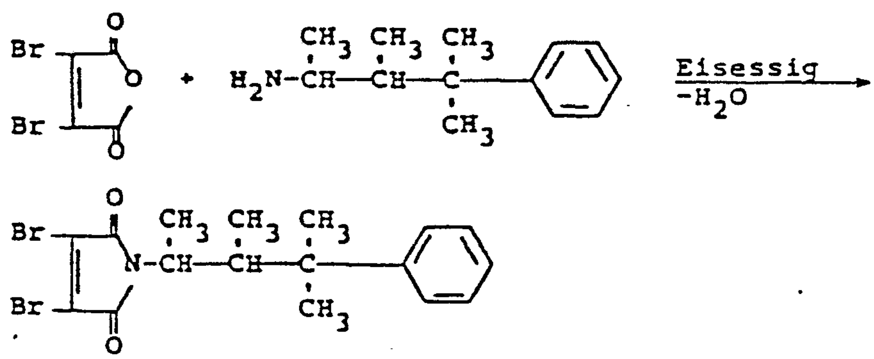


Verwendet man bei der Verfahrensvariante b) Dichlormaleinsäuredimethylester und 2-Phenyl-1,2-dimethylethylamin als Ausgangsstoffe, so kann der Reaktions-verlauf durch das folgende Formelschema wiedergegeben werden:

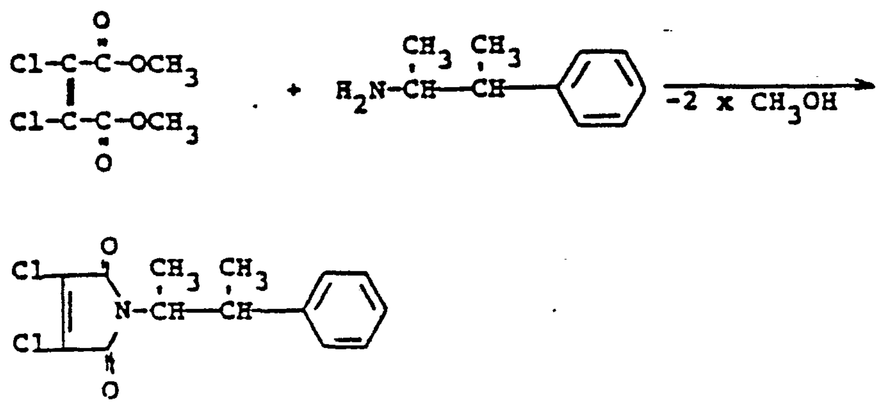


Verwendet man bei der Verfahrensvariante c) Dichlormaleinsäureanhydrid, 1,1-Dimethyl-2-phenylethylamin als Ausgangsstoffe, so erhält man die Dichlormaleinsäuremonoamide, die cyclisiert werden. Dieser Reaktions-verlauf kann durch das folgende Formelschema wiedergegeben werden:

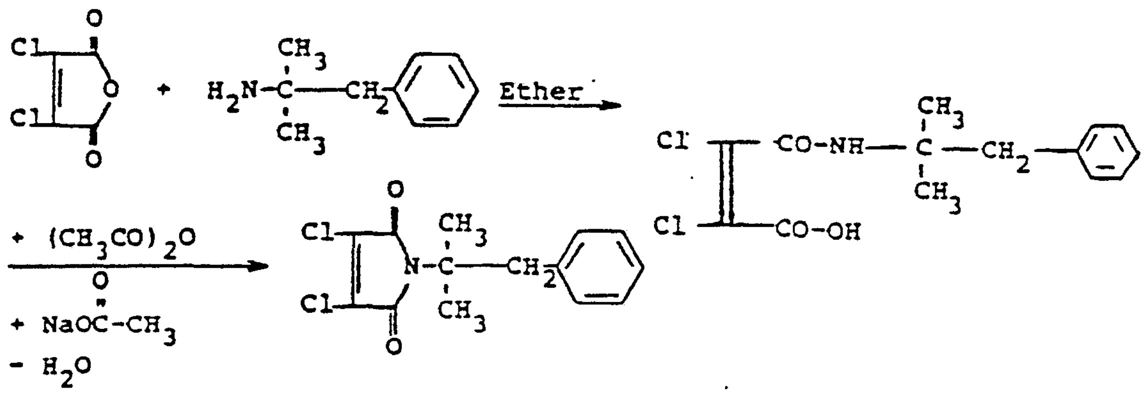


Die bei der Durchführung der Verfahrensvariante a) als Ausgangsverbindungen benötigten Halogenmaleinsäureanhydride sind durch die Formel (II) allgemin definiert. Diese Verbindungen sind im Handel erhältlich und/oder nach bekannten Verfahren leicht herstellbar. Die weiterhin bei den Verfahrensvarianten a) und b) einzusetzenden Amine sind durch die Formel (III) definiert. In dieser Formel haben die Reste, die bereits im Zusammenhang mit der Beschreibung der erfindungsgemäßen Stoffe der Formel (I) gegebene Bedeutung. Die Amine sind teilweise bekannt oder können nach allgemein bekannten Verfahren hergestellt werden. So können die Amine beispielsweise durch Reduktion von Nitrilen oder Ketoximen mit Wasserstoff hergestellt werden (vgl. Houben-Weyl, Methoden der organischen Chemie, XI/1, Seiten 341 ff). Weiterhin ist eine gängige Methode die reduktive Aminierung von Aldehyden oder Ketonen mit Wasserstoff und Ammoniak (vgl. Houben-Weyl, XI/1, Seite 341). Zur Herstellung von Aralkylaminen eignet sich vor allem die Methode der Aminierung von Halogeniden (vgl. Houben-Weyl, Bd. XI/1, Seite 24).

Die bei der Verfahrensvariante b) noch benötigten Halogenmaleinsäuredialkylester sind durch die Formel (IV) beschrieben. Diese Ester sind bekannt und nach gängigen Verfahren aus den käuflichen Halogenmaleinsäureanhydriden durch Umsetzung mit Alkoholen erhältlich.

Die bei der Verfahrensvariante c) einzusetzenden Halogenmaleinsäuremonoamide der Formel (V) sind teilweise bekannt oder können nach an sich bekannten Verfahren aus den entsprechenden Maleinsäureanhydriden durch Umsetzung mit primären Aminen hergestellt werden (vgl. Organic Synthesis 41, Seite 93 (1961)).

Als Verdünnungsmittel kommen für die Verfahrensvariante a) vor allem Carbonsäuren in Frage, wie z.B. Ameisensäure, Essigsäure und Propionsäure.

Als Verdünnungsmittel kommen bei der Verfahrensvariante b) organische Lösungsmittel in Frage. Hierzu gehören vorzugsweise Toluol, Xylol, Chlorbenzol, Perchlorethan, Dioxan, Glykoldimethylether, Dimethylformamid.

Bei der Verfahrensvariante c) werden vorzugsweise als Verdünnungsmittel eingesetzt: Carbonsäuren, wie Essigsäure; aromatische Kohlenwasserstoffe, wie Toluol, Xylol; Halogenkohlenwasserstoffe, wie Chlorbenzol; weiterhin Dioxan und als anhydridisierende Reagenzien vorzugsweise Acetanhydrid, Phosgen, Thionylchlorid, Phosphoroxichlorid, Phosphorpentachlorid.

Die Reaktionstemperaturen können bei der Durchführung der verschiedenen Verfahrensvarianten in einem größeren Bereich variiert werden. Bei Verfahrensvariante a) arbeitet man bei Temperaturen von 20 bis 150°C, vorzugsweise von 80 bis 120°C. Bei Verfahrensvariante b) arbeitet man bei Temperaturen von 50 bis 180°C, vorzugsweise von 80 bis 130°C und bei der Verfahrensvariante c) bei Temperaturen von 0 bis 150°C, vorzugsweise von 50 bis 120°C.

Bei allen drei Varianten wird im allgemeinen bei Normaldruck gearbeitet.

Bei der Durchführung aller Verfahrensvarianten werden die Ausgangsstoffe vorzugsweise in äquimolaren Mengen eingesetzt.

Nach einer bevorzugten Ausführungsform der Verfahrensvariante a) werden die Ausgangsstoffe in äquimolaren Mengen in einem organischen Lösungsmittel, z.B. Eisessig mehrere Stunden bei erhöhter Temperatur gerührt. Anschließend auf Raumtemperatur abgekühlt, Wasser wird zugesetzt, wobei das Produkt bereits ausgeschieden wird.

Setzt man das Dibrommaleinsäureanhydrid als Ausgangstoff ein, so wird dieses in einer bevorzugten Ausführungsform in der Lösung aus Dibrommaleinsäure in Eisessig unter Rühren hergestellt und in dieser Lösung direkt weiter mit dem Amin umgesetzt.

Nach einer bevorzugten Ausführungsform der Verfahrensvariante b) wird der Dihalogenmaleinsäuredialkylester aus Dihalogenmaleinsäureanhydrid und Methanol hergestellt und dieser nach fraktionierter Destillation mit dem Amin umgesetzt. Die Aufarbeitung erfolgt wie oben beschrieben (vgl. US-Patent 3 734 927, Beispiel 5).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für

den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobaceteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

In entsprechenden Konzentrationen angewendet sind die Verbindungen auch akarizid wirksam.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mittelen. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hoch-disperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-% vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Herstellungsbeispiele

Beispiel 1

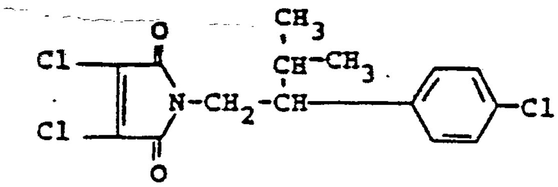

16,7 g (0,1 mol) Dichlormaleinsäureanhydrid und 19,7 g (0,1 mol) 2-(4-Chlorphenyl)-3-methylbutylamin werden in 100 ml Eisessig 4 Stunden bei 120°C gerührt. Man kühlt auf 20°C ab und gibt 10 ml Wasser zu. Es fällt ein farbloser Niederschlag aus, der abgesaugt und getrocknet wird. Man erhält 21,2 g Dichlormaleinsäure-N-[2-(4-chlorphenyl)-3-methylbutyl]-imid mit dem Schmelzpunkt 91—92°C. Durch Verrühren der Mutterlauge mit Wasser können noch weitere 10,4 g Imid isoliert werden. Die Gesamtausbeute beträgt: 91,3% der Theorie.

Beispiel 2

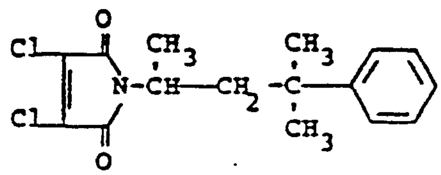

16,7 g (0,1 mol) Dichlormaleinsäureanhydrid und 17,7 g (0,1 mol) 3-Phenyl-1,3,3-trimethylpropylamin werden in 100 ml Eisessig 4 Stunden am Rückfluß gerührt. Man gibt 50 ml Wasser zu und kühlt auf 20°C ab. Das Produkt wird abgesaugt und getrocknet. Man erhält 23,5 g (72% der Theorie) Dichlormaleinsäure-N-[3-phenyl-1,3,3-trimethylpropyl]-imid mit einem Schmelzpunkt von 106—109°C.

Beispiel 3

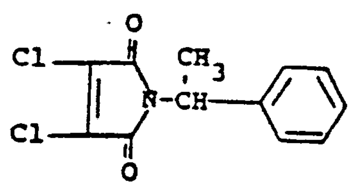

16,7 g (0,1 mol) Dichlormaleinsäureanhydrid und 12,0 g (0,1 mol) α-Methylbenzylamin werden in 100 ml Eisessig 4 Stunden zum Sieden erhitzt. Dann werden 100 ml Wasser zugesetzt und das ausgefallene Öl mit Methylenchlorid extrahiert. Die Methylenchloridphase wird abgetrennt, mit Wasser gewaschen, getrocknet und eingedampft. Das verbleibende Öl kristallisiert. Man erhält 20,8 (77,3% der Theorie) Dichlormaleinsäure-N-1-phenylethylimid mit dem Schmelzpunkt 62—65°C.

Beispiel 4

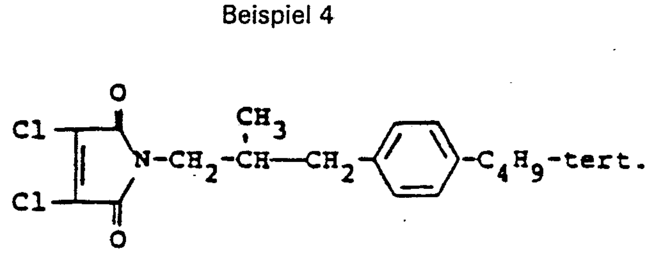

16,7 g (0,1 mol) Dichlormaleinsäureanhydrid und 20,5 g (0,1 mol) 2-Methyl-3-(4-tert.-butylphenyl)-propylamin werden in 100 ml Eisessig 4 Stunden bei Rückfluß gerührt. Man gibt 100 ml Wasser zu, extrahiert das ausgefallene Öl mit Methylenchlorid, engt die Methylenchlorid-phase ein und kristallisiert den Rückstand aus Ethanol um. Man erhält 14,6 g (41,2% der Theorie) Dichlormaleinsäure-N-[2-methyl-3-(4-tert.-butylphenyl)-propyl]-imid mit dem Schmelzpunkt 64—65°C.

Beispiel 5

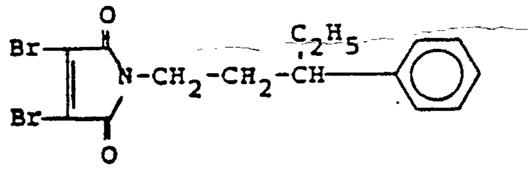

14 g (0,051 mol) Dibrommaleinsäure werden in 100 ml Eisessig 1 Stunde bei Rückfluß gerührt. In die erkaltete Lösung des so hergestellten Dibrommaleinsäureanhydrids gibt man 8,2 g (0,05 mol) 3-Ethyl-3-phenyl-propylamin und rührt weitere 3 Stunden bei Rückfluß. Man kühlt auf 20°C und saugt den Niederschlag ab. Nach Trocknen erhält man 11,4 g (56% der Theorie) Dibrommaleinsäure-N-3-phenylpentylimid mit dem Schmelzpunkt: 71—73°C.

Analog einem der unter 1—5 beschriebenen Beispiele können die nachfolgend aufgeführten Verbindungen der Formel (I) hergestellt werden:

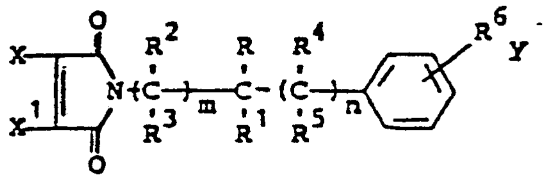

(I)

| Bsp. Nr. | X | $X^1$ | R | $R^1$ | m | $R^2$ | $R^3$ | n | $R^4$ | $R^5$ | $R^6$ | Y | Schmelzpunkt (°C) |
|---|---|---|---|---|---|---|---|---|---|---|---|---|---|
| 6 | Cl | Cl | $CH_3$ | H | 1 | H | H | 0 | – | – | – | 0 | 89–90 |
| 7 | Cl | Cl | $CH_3$ | $CH_3$ | 1 | H | H | 0 | – | – | – | 0 | |
| 8 | Cl | Cl | $C_6H_5$ | H | 0 | – | – | 1 | H | H | – | 0 | 88–89 |
| 9 | Cl | Cl | $C_6H_5$ | H | 1 | H | H | 0 | – | – | – | 0 | 132–133 |
| 10 | Cl | Cl | $CH_2-C_6H_5$ | H | 0 | – | – | 1 | H | H | – | 0 | 123–124 |
| 11 | Cl | Cl | $CH_3$ | H | 1 | H | H | 0 | – | – | 2-Cl | 1 | 94–95 |
| 12 | Cl | Cl | $CH_3$ | H | 1 | H | H | 0 | – | – | 4-Cl | 1 | |
| 13 | Cl | Cl | $CH_3$ | $CH_3$ | 1 | H | H | 0 | – | – | 4-Cl | 1 | 98–99 |
| 14 | Cl | Cl | iso-$C_3H_7$ | H | 1 | H | H | 0 | – | – | 4-Cl | 1 | 91–92 |
| 15 | Cl | Cl | $CH_3$ | H | 1 | H | H | 0 | – | – | 2,4-$Cl_2$ | 2 | 118–119 |
| 16 | Cl | Cl | $CH_3$ | H | 1 | H | H | 0 | – | – | 3,4-$Cl_2$ | 2 | |
| 17 | Cl | Cl | $CH_3$ | $CH_3$ | 1 | H | H | 0 | – | – | 3,4-$Cl_2$ | 2 | 98–99 |
| 18 | Cl | Cl | $CH_3$ | H | 1 | H | H | 0 | – | – | 2,6-$Cl_2$ | 2 | 86 |
| 19 | Cl | Cl | $C_6H_5-CH_2-$ | H | 1 | H | H | 0 | – | – | 2,6-$Cl_2$ | 2 | 108–109 |
| 20 | Cl | Cl | $CH_3$ | H | 0 | – | – | 0 | – | – | – | 0 | 62–65 |
| 21 | Cl | Cl | $CH_3$ | H | 0 | – | – | 0 | – | – | 4-$CH_3$ | 1 | 79–80 |
| 22 | Cl | Cl | $CH_3$ | H | 0 | – | – | 0 | – | – | tert.-$C_4H_9$ | 1 | Öl |
| 23 | Cl | Cl | $CH_3$ | H | 1 | H | H | 1 | H | H | – | 0 | 88–89 |

| Bsp. Nr. | X | $X^1$ | R | $R^1$ | m | $R^2$ | $R^3$ | n | $R^4$ | $R^5$ | $R^6$ | Y | Schmelzpunkt (°C) |
|---|---|---|---|---|---|---|---|---|---|---|---|---|---|
| 24 | Cl | Cl | n-$C_3H_7$ | H | 0 | – | – | 0 | – | – | – | 0 | Öl |
| 25 | Cl | Cl | $CH_3$ | H | 1 | H | H | 1 | H | H | 2-Cl | 1 | 98–100 |
| 26 | Cl | Cl | $CH_3$ | H | 0 | – | – | 0 | – | – | 4-Cl | 1 | 101–102 |
| 27 | Cl | Cl | $CH_3$ | H | 0 | – | – | 0 | – | – | 4-$OCH_3$ | 1 | Öl |
| 28 | Cl | Cl | $CH_3$ | H | 0 | – | – | 0 | – | – | 3-$CF_3$ | 1 | |
| 29 | Cl | Cl | $CH_3$ | H | 1 | H | H | 1 | H | H | 4-Cl | 1 | |
| 30 | Cl | Cl | $CH_3$ | H | 1 | H | H | 1 | H | H | 2,4-$Cl_2$ | 2 | 85 |
| 31 | Cl | Cl | $CH_3$ | H | 1 | H | H | 1 | H | H | 3,4-$Cl_2$ | 2 | |
| 32 | Cl | Cl | $CH_3$ | H | 1 | H | H | 1 | H | H | 2,6-$Cl_2$ | 2 | 82–83 |
| 33 | Cl | Cl | $CH_3$ | H | 1 | H | H | 1 | H | H | 4-$CH_3$ | 1 | 99–101 |
| 34 | Cl | Cl | $CH_3$ | H | 1 | H | H | 1 | H | H | 4-$OCH_3$ | 1 | 106–107 |
| 35 | Cl | Cl | $CH_3$ | H | 1 | H | H | 1 | H | H | 4-tert.$C_4H_9$ | 1 | 64–65 |
| 36 | Cl | Cl | $CH_3$ | H | 0 | – | – | 2 | H | H | – | 0 | Öl |
| 37 | Cl | Cl | iso-$C_4H_9$ | H | 0 | – | – | 2 | H | H | – | 0 | Öl |
| 38 | Cl | Cl | $CH_3$ | H | 0 | – | – | 2 | H | H | 2-Cl | 1 | |
| 39 | Cl | Cl | iso-$C_4H_9$ | H | 0 | – | – | 2 | H | H | 2-Cl | 1 | Öl |
| 40 | Cl | Cl | $CH_3$ | H | 0 | – | – | 2 | H | H | 4-Cl | 1 | |
| 41 | Cl | Cl | iso-$C_4H_9$ | H | 0 | – | – | 2 | H | H | 4-Cl | 1 | Öl |

| Bsp. Nr. | X | $X^1$ | R | $R^1$ | m | $R^2$ | $R^3$ | n | $R^4$ | $R^5$ | $R^6$ | Y | Schmelzpunkt (°C) |
|---|---|---|---|---|---|---|---|---|---|---|---|---|---|
| 42 | Cl | Cl | $CH_3$ | H | 0 | – | – | 2 | H | H | 4-$CH_3$ | 1 | Öl |
| 43 | Cl | Cl | $CH_3$ | H | 0 | – | – | 2 | H | H | 3,4-$Cl_2$ | 2 | |
| 44 | Cl | Cl | $CH_3$ | H | 0 | – | – | 2 | H | H | 4-$OCH_3$ | 1 | |
| 45 | Cl | Cl | $CH_3$ | H | 2 | H | H | 0 | – | – | – | 0 | |
| 46 | Cl | Cl | $CH_3$ | H | 2 | H | H | 0 | – | – | 2-Cl | 1 | |
| 47 | Cl | Cl | $CH_3$ | H | 2 | H | H | 0 | – | – | 4-Cl | 1 | |
| 48 | Cl | Cl | $CH_3$ | H | 2 | H | H | 0 | – | – | 3,4-$Cl_2$ | 2 | |
| 49 | Cl | Cl | $CH_3$ | H | 2 | H | H | 0 | – | – | 4-$CH_3$ | 1 | |
| 50 | Cl | Cl | $CH_3$ | H | 2 | H | H | 0 | – | – | 4-$OCH_3$ | 1 | |
| 51 | Cl | Cl | $C_2H_5$ | H | 2 | H | H | 0 | – | – | – | 0 | Öl |
| 52 | Cl | Cl | $CH_3$ | H | 1 | $CH_3$ | H | 1 | H | H | – | 0 | Öl |
| 53 | Cl | Cl | $CH_3$ | H | 1 | $CH_3$ | H | 1 | H | H | 3-Cl | 1 | Öl |
| 54 | Cl | Cl | $CH_3$ | H | 0 | – | – | 2 | H<br>$CH_3$ | H u.<br>$CH_3$ | – | 0 | 106–109 |
| 55 | Cl | Cl | $CH_3$ | H | 0 | – | – | 2 | H<br>$CH_3$ | H u.<br>$CH_3$ | 4-Cl | 1 | 113–114 |
| 56 | Br | Br | $CH_3$ | H | 1 | H | H | 1 | H | H | 4-t-$C_4H_9$ | 1 | 118–119 |
| 57 | Br | Br | $C_2H_5$ | H | 2 | H | H | 0 | – | – | – | 0 | |
| 58 | Br | Br | i-$C_4H_9$ | H | 0 | – | – | 0 | H | H | 2-Cl | 1 | Öl |

| Bsp. Nr. | X | $X^1$ | R | $R^1$ | m | $R^2$ | $R^3$ | n | $R^4$ | $R^5$ | $R^6$ | Y | Schmelzpunkt (°C) |
|---|---|---|---|---|---|---|---|---|---|---|---|---|---|
| 59 | Br | Br | $CH_3$ | $CH_3$ | 2 | $CH_3$ u.R | H H | 0 | – | – | 4-Cl | 1 | 106–108 |
| 60 | Cl | H | $CH_3$ | H | 1 | H | H | 1 | H | H | 3,4-$Cl_2$ | 2 | |
| 61 | Cl | Cl | $CH_3$ | H | 1 | $CH_3$ | H | 1 | $CH_3$ | H | – | 0 | |
| 62 | Cl | Cl | $CH_3$ | H | 1 | H | H | 1 | $CH_3$ | H | – | 0 | |

Anwendungsbeispiele

In den nachfolgenden Beispielen werden die nachstehend angegebenen Verbindungen als Vergleichssubstanz eingesetzt:

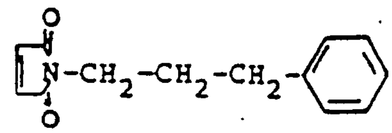

(A)

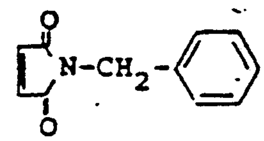

(B)

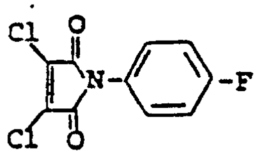

(C)

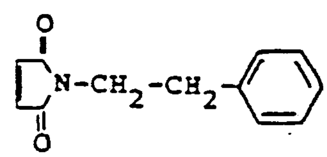

(D)

Beispiel A

Leptosphaeria nodorum-Test (Weizen)/protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 6 und 4.

Beispiel B

Puccinia-Test (Weizen)/protektiv
Lösungsmittel: 100 Gew.-Teile Dimethylformamid
Emulgator: 0,25 Gew.-Teile Alkyl-a-ryl-Polyglykol-ether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 55, 41, 22, 3 und 1.

Beispiel C

Venturia-Test (Apfel)/protektiv
Lösungsmittel: 4,7 Gew.-Teile Aceton
Emulgator: 0,3 Gew.-Teile Alkyl-a-ryl-polyglykol-ether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativer Luftfeuchtigkeit von ca. 70% aufgestellt.

12 Tage nach den Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 6, 4, 55, 41, 22 und 53.

Beispiel D

Pyricularia-Test (Reis)/protektiv
Lösungsmittel: 12,5 Gew.-Teile Aceton
Emulgator: 0,3 Gew-Teile Alkyla-rylpolyglykolether

Zur Herstellung einer zweckmäßigen Wirkstoffzubereitung vermischt man 1 Gew.-Teil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bie zur Tropfnässe. Nach Antrocknen des Spritzbelages werden die Pflanzen mit einer wäßrigen Sporensuspension von Pyricularia oryzae inokuliert. Anschließend werden die Pflanzen in einem Gewächshaus bei 100% relativer Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z.B. die Verbindungen gemäß folgender Herstellungsbeispiele: 10 und 14.

Beispiel E

Pyricularia-Test (Reis)/systemisch
Lösungsmittel: 12,5 Gew.-Teile Aceton
Emulgator: 0,3 Gew.-Teile Alkyla-rylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer relativen Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigt bei diesem Test z.B. die Verbindung gemäß folgendem Herstellungsbeispiel: 4

Beispiel F

Pellicularia-Test (Reis)
Lösungsmittel: 12,5 Gew.-Teile Aceton
Emulgator: 0,3 Gew.-Teil Alkyla-rylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstudium tropfnaß gespritzt. Die Pflanzen verbleiben bis zum Abtrocknen im Gewächshaus. Anschließend werden die Pflanzen mit Pellicularia sasakii inokuliert und bei 25°C und 100% relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigt bei diesem Test z.B. die Verbindung gemäß folgendem Herstellungsbeispiel: 55.

**Patentansprüche**

1. Substituierte Maleinsäureimide der Formel (I)

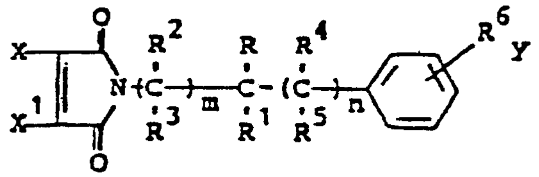

(I)

in welcher

X und $X^1$ für Chlor,

R für geradkettiges oder verzweigtes Alkyl mit 1 bis 4 Kohlenstoffatomen, wie Methyl, Ethyl, n-Propyl, iso-Propyl, n-Butyl, sec.-Butyl, iso-Butyl und tert.-Butyl, für gegebenenfalls ein- bis dreifach, gleich oder verschieden durch Methyl, Ethyl, Chlor und Brom substituiertes Phenyl, Benzyl bzw. Phenethyl,

$R^1$ bis $R^5$ gleich oder verschieden sind und für Wasserstoff, geradkettiges oder verzweigtes Alkyl mit 1 bis 4 Kohlenstoffatomen, wie Methyl, Ethyl, n-Propyl, iso-Propyl, n-Butyl, iso-Butyl, sec.-Butyl und tert.-Butyl, für gegebenenfalls ein- bis dreifach, gleich oder verschieden durch Methyl, Ethyl und Chlor substituiertes Phenyl, Benzyl bzw. Phenethyl,

$R^6$ für Chlor, Brom, Alkyl mit 1 bis 4 Kohlenstoffatomen, wie Methyl, Ethyl, n-Propyl, iso-Propyl, n-Butyl, sec.-Butyl, iso-Butyl und tert.-Butyl, für Alkoxy mit 1 bis 3 Kohlenstoffatomen, wie Methoxy, Ethoxy, n-Propoxy und iso-Propoxy, für Sulfonylamin, Alkylsulfonyl bzw. Alkylcarbonyl mit 1 bis 3 Kohlenstoffatomen im Alkylteil, wie Methylsulfonyl, Ethylsulfonyl, n-Propylsulfonyl, iso-Propylsulfonyl, Methylcarbonyl, Ethylcarbonyl, n-Propylcarbonyl und iso-Propylcarbonyl, für Carbonylamin, Trifluormethyl, Phenyl, Cyclopentyl oder Cyclohexyl,

m und n für 0, 1, 2 oder 3 stehen, die Summe aus

n und m für 0, 1 oder 2 und

y für 0, 1, 2 oder 3 stehen, ausgenommen die Verbindungen, in denen

X, $X^1$ für Chlor,

m und y für 0

n für 1

$R^1$, $R^4$ und $R^5$ für Wasserstoff und

R für Methyl und

X, $X^1$ für Chlor,

m, n und y für 0, $R^1$ für Wasserstoff und R für Phenyl stehen.

2. Verfahren zur Herstellung von substituierten Maleinsäureimiden der Formel (I)

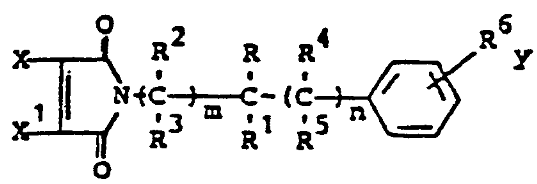

(I)

in welcher

X und $X^1$ fur Chlor,

R für geradkettiges oder verzweigtes Alkyl mit 1 bis 4 Kohlenstoffatomen, wie Methyl, Ethyl, n-Propyl, iso-Propyl, n-Butyl, sec.-Butyl, iso-Butyl und tert.-Butyl, für gegebenenfalls ein- bis dreifach, gleich oder verschieden durch Methyl, Ethyl, Chlor und Brom substituiertes Phenyl, Benzyl bzw. Phenethyl,

$R^1$ bis $R^5$ gleich oder verschieden sind und für Wasserstoff, geradkettiges oder verzweigtes Alkyl mit 1 bis 4 Kohlenstoffatomen, wie Methyl, Ethyl, n-Propyl, iso-Propyl, n-Butyl, iso-Butyl, sec.-Butyl und tert.-Butyl, für gegebenenfalls ein- bis dreifach, gleich oder verschieden durch Methyl, Ethyl und Chlor substituiertes Phenyl, Benzyl bzw. Phenethyl,

$R^6$ für Chlor, Brom, Alkyl mit 1 bis 4 Kohlenstoffatomen, wie Methyl, Ethyl, n-Propyl, iso-Propyl, n-Butyl, sec.-Butyl, iso-Butyl und tert.-Butyl, für Alkoxy mit 1 bis 3 Kohlenstoffatomen, wie Methoxy, Ethoxy, n-Propoxy und iso-Propoxy, für Sulfonylamin, Alkylsulfonyl bzw. Alkylcarbonyl mit 1 bis 3 Kohlenstoffatomen im Alkylteil, wie Methylsulfonyl, Ethylsulfonyl, n-Propylsulfonyl, iso-Propylsulfonyl, Methylcarbonyl, Ethylcarbonyl, n-Propylcarbonyl und iso-Propylcarbonyl, für Carbonylamin, Trifluormethyl, Phenyl, Cyclopentyl oder Cyclohexyl,

m und n für 0, 1, 2 oder 3 stehen, die Summe aus

n und m für 0, 1 oder 2 und

y für 0, 1, 2 oder 3 stehen, ausgenommen die Verbindungen, in denen
X, $X^1$ für Chlor,
m und Y für O,
n für 1,
$R^1$, $R^4$ und $R^5$ für Wasserstoff und
R für Methyl stehen und
X, $X^1$ für Chlor,
m, n und y für 0 und
$R^1$ für Wasserstoff und
R für Phenyl stehen, dadurch gekennzeichnet, daß man
a) ein Halogenmaleinsäureanhydrid der Formel (II)

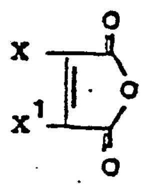
(II)

in welcher
X und $X^1$ die oben angegebene Bedeutung haben, mit primären Aminen der Formel (III)

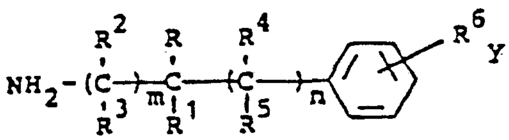
(III)

in welcher
R bis $R^6$, m, n und y die oben angegebene Bedeutung haben,
in einem Verdünnungsmittel umsetzt, oder
b) einen Halogenmaleinsäuredialkylester der Formel (IV)

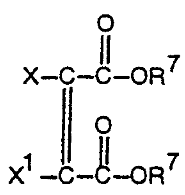
(IV)

in welcher
X und $X^1$ die oben angegebene Bedeutung haben, und
$R^7$ für Alkyl steht,
mit primären Aminen der Formel (III), in welcher
R bis $R^6$, m, n und y die oben angegebene Bedeutung haben,
gegebenenfalls in einem Lösungs- oder Verdünnungsmittel umsetzt, oder
c) Halogenmaleinsäuremonoamide der Formel (V)

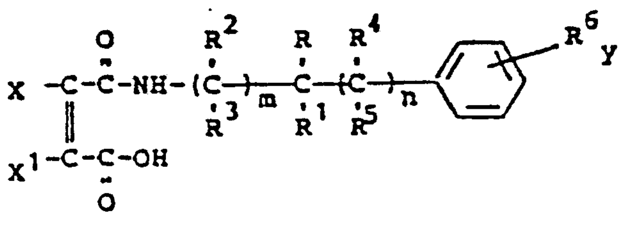
(V)

in welcher
X, $X^1$, R bis $R^6$, m, n und y die oben angegebene Bedeutung haben,
in Gegenwart eines Lösungsmittels, wie z.B. Eisessig und gegebenenfalls eines anhydridisierenden Mittels, wie z.B. Acetanhydrid oder Thionylchlorid zu den Verbindungen der Formel (I) cyclisiert.

3. Dibrommaleinsäure-N-[2-methyl-3-(4-tert.-butyl-phenyl)-propyl]-imid.
4. Dibrommaleinsäure-N-3-phenyl-pentylimid.
5. Dibrommaleinsäure-N-[1-methyl-3-methyl-3-(4-chlorphenyl)-butyl]-imid.
6. Schädlingsbekämpfungsmittel, gekennzeichnet durch einen Gehalt an mindestens einem substituierten Maleinsäureimid der Formel (I) gemäß Ansprüchen 1 bis 5.
7. Verwendung von substituierten Maleinsäureimiden der Formel (I) gemäß den Ansprüchen 1 bis 5 zur Bekämpfung von Schädlingen.
8. Verfahren zur Bekämpfung von Schädlingen, dadurch gekennzeichnet, daß man substituierte Maleinsäureimide der Formel (I) gemäß den Ansprüchen 1 bis 5 auf Schädlinge und/oder ihren Lebensraum einwirken läßt.
9. Verfahren zur Herstellung von Schädlingsbekämpfungsmitteln, dadurch gekennzeichnet, daß man substituierte Maleinsäureimide der Formel (I) gemäß den Ansprüchen 1 bis 5 mit Streckmitteln und/oder oberflächenaktiven Mitteln vermischt.

**Claims**

1. Substituted maleic acid imides of the formula (I)

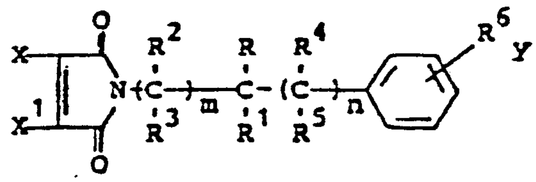

(I)

in which
X and $X^1$ represent chlorine,
R represents straight-chain or branched alkyl with 1 to 4 carbon atoms, such as methyl, ethyl, n-propyl, iso-propyl, n-butyl, sec.-butyl, iso-butyl and tert.-butyl, or phenyl, benzyl or phenethyl, which are optionally mono- to tri-substituted by identical or different substituents from the group comprising methyl, ethyl chlorine and bromine,
$R^1$ to $R^5$ are identical or different and represent hydrogen, straight-chain or branched alkyl with 1 to 4 carbon atoms, such as methyl, ethyl, n-propyl, iso-propyl, n-butyl, iso-butyl, sec.-butyl and tert.-butyl, or phenyl, benzyl or phenethyl which are optionally mono- to tri-substituted by identical or different substituents from the group comprising methyl, ethyl and chlorine,
$R^6$ represents chlorine, bromine, alkyl with 1 to 4 carbon atoms, such as methyl, ethyl, n-propyl, iso-propyl, n-butyl, sec.-butyl, iso-butyl and tert.-butyl, alkoxy with 1 to 3 carbon atoms, such as methoxy, ethoxy, n-propoxy and iso-propoxy, sulphonylamine, alkylsulphonyl or alkylcarbonyl with 1 to 3 carbon atoms in the alkyl part, such as methylsulphonyl, ethylsulphonyl, n-propylsulphonyl, iso-propylsulphonyl, n-methylcarbonyl, ethylcarbonyl, n-propylcarbonyl and iso-propylcarbonyl, carbonylamine, trifluoromethyl, phenyl, cyclopentyl or cyclohexyl,
m and n represent 0, 1, 2 or 3, the sum of
n and m represent 0, 1 or 2 and
y represents 0, 1, 2 or 3, with the exception of those compounds in which
X and $X^1$ represents chlorine,
m and y represent 0,
n represents 1,
$R^1$, $R^4$ and $R^5$ represent hydrogen and
R represents methyl and
X and $X^1$ represent chlorine,
m, n and y represent 0, $R^1$ represents hydrogen and R represents phenyl.

2. Process for the preparation of substituted maleic acid imides of the formula (I)

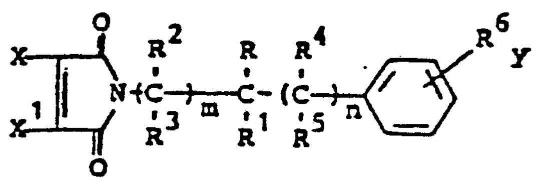

(I)

in which
X and $X^1$ represent chlorine,
R represents straight-chain or branched alkyl with 1 to 4 carbon atoms, such as methyl, ethyl, n-propyl, iso-propyl, n-butyl, sec.-butyl, iso-butyl and tert.-butyl, or phenyl, benzyl or phenethyl, which are optionally

mono- to tri-substituted by identical or different substituents from the group comprising methyl, ethyl, chlorine and bromine, $R^1$ to $R^5$ are identical or different and represent hydrogen, straight-chain or branched alkyl with 1 to 4 carbon atoms, such as methyl, ethyl, n-propyl, iso-propyl, n-butyl, iso-butyl, sec.-butyl and tert.-butyl, or phenyl, benzyl or phenethyl which are optionally mono- to tri-substituted by identical or different substituents from the group comprising methyl, ethyl and chlorine,

$R^6$ represents chlorine, bromine, alkyl with 1 to 4 carbon atoms, such as methyl, ethyl, n-propyl, iso-propyl, n-butyl, sec.-butyl, iso-butyl and tert.-butyl, alkoxy with 1 to 3 carbon atoms, such as methoxy, ethoxy, n-propoxy and iso-propoxy, sulphonylamine, alkylsulphonyl or alkylcarbonyl with 1 to 3 carbon atoms in the alkyl part, such as methylsulphonyl, ethylsulphonyl, n-propylsulphonyl, iso-propylsulphonyl, methylcarbonyl, ethylcarbonyl, n-propylcarbonyl and iso-propylcarbonyl, carbonylamine, trifluoromethyl, phenyl, cyclopentyl or cyclohexyl,

m and n represent 0, 1, 2 or 3, the sum of
n and m represents 0, 1 or 2 and
y represents 0, 1, 2 or 3, with the exception of those compounds in which
X and $X^1$ represents chlorine,
m and Y represent 0,
n represents 1,
$R^1$, $R^4$ and $R^5$ represent hydrogen and
R represents methyl and
X and $X^1$ represent chlorine,
m, n and y represent 0 and
$R^1$ represents hydrogen and
R represents phenyl,

characterised in that

a) a halogenomaleic acid anhydride of the formula (II)

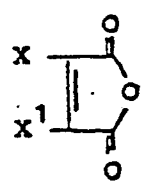

(II)

in which

X and $X^1$ have the abovementioned meaning, is reacted with primary amines of the formula (III)

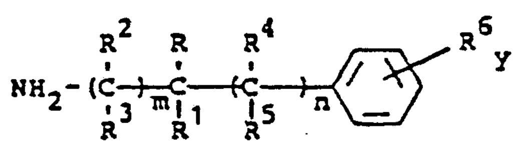

(III)

in which

R to $R^6$, m, n and y have the abovementioned meaning, in a diluent, or

b) a halogenomaleic acid dialkyl ester of the formula (IV)

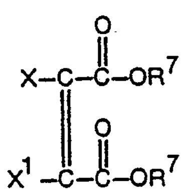

(IV)

in which

X and $X^1$ have the abovementioned meaning, and
$R^7$ represents alkyl,

is reacted with primary amines of the formula (III), in which

R to $R^6$, m, n and y have the abovementioned meaning, if appropriate in a solvent or diluent, or

c) halogenomaleic acid monoamides of the formula (V)

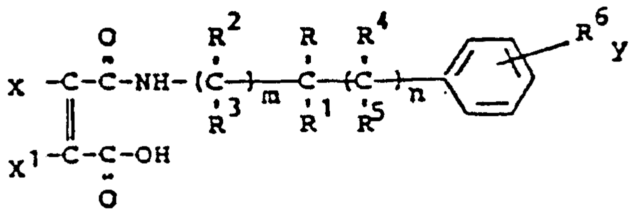

(V)

in which

X, $X^1$, R to $R^6$, m, n and y have the abovementioned meaning, are cyclised in the presence of a solvent, such as, for example, glacial acetic acid, and, if appropriate, an anhydrising agent, such as, for example, acetic anhydride or thionyl chloride, to give the compounds of the formula (I).

3. Dibromomaleic acid N-[2-methyl-3-(4-tert.-butyl-phenyl)-propyl]-imide.

4. Dibromomaleic acid N-3-phenyl-pentylimide.

5. Dibromomaleic acid N-[1-methyl-3-methyl-3-(4-chlorophenyl)-butyl]-imide.

6. Agents for combating pests, characterised in that they contain at least one substituted maleic acid imide of the formula (I) according to Claims 1 to 5.

7. Use of substituted maleic acid imides of the formula (I) according to Claims 1 to 5 for combating pests.

8. Method of combating pests, characterised in that substituted maleic acid imides of the formula (I) according to Claims 1 to 5 are allowed to act on pests and/or their environment.

9. Process for the preparation of agents for combating pests, characterised in that substituted maleic acid imides of the formula (I) according to Claims 1 to 5 are mixed with extenders and/or surface-active agents.

**Revendications**

1. Imides d'acides maléiques substitués de formule (I)

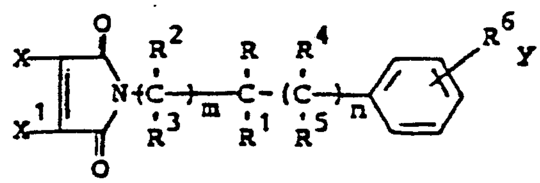

(I)

dans laquelle

X et $X^1$ représentent du chlore,

R est un groupe alkyle à chaîne droite ou ramifié ayant 1 à 4 atomes de carbone, tel que méthyle, éthyle, n-propyle, isopropyle, n-butyle, sec.-butyle, isobutyle et tertiobutyle, un groupe phényle, benzyle ou phénéthyle portant éventuellement un à trois substituants méthyle, éthyle, chloro et bromo égaux ou différents,

$R^1$ à $R^5$ sont identiques ou différents et représentent de l'hydrogène, un groupe alkyle à chaîne droite ou ramifié ayant 1 à 4 atomes de carbone, tel que méthyle, éthyle, n-propyle, isopropyle, n-butyle, isobutyle, sec.-butyle et tertio-butyle, un groupe phényle, benzyle ou phénéthyle portant éventuellement un à trois substituants méthyle, éthyle et chloro égaux ou différents,

$R^6$ désigne du chlore, du brome, un groupe alkyle ayant 1 à 4 atomes de carbone tel que méthyle, éthyle, n-propyle, isopropyle, n-butyle, sec.-butyle, isobutyl et tertio-butyle, un groupe alkoxy ayant 1 à 3 atomes de carbone, tel que méthoxy, éthoxy, n-propoxy et isopropxy, un groupe sulfonylamino, alkylsulfonyle ou alkylcarbonyle ayant 1 à 3 atomes de carbone dans la partie alkyle, tel que méthylsulfonyle, éthylsulfonyle, n-propylsulfonyle, isopropylsulfonyle, méthylcarbonyle, éthylcarbonyle, n-propylcarbonyle et isopropylcarbonyle, un groupe carbonylamino, trifluorométhyle, phényle, cyclopentyle ou cyclohexyle,

*m* et *n* sont égaux à 0, 1, 2 ou 3, la somme de
*n* et *m* étant égale à 0, 1 ou 2 et
Y a la valeur 0, 1, 2 ou 3, excepté les composés dans lesquels
X, $X^1$ représentent du chlore,
*m* et *y* sont égaux à 0
*n* a la valeur 1

$R^1$, $R^4$ et $R^5$ représentent de l'hydrogène et R est un groupe méthyle, et les composés dans lesquels X, $X^1$ représentent du chlore, *m*, *n* et *y* sont égaux à 0, $R^1$ est de l'hydrogène et R est un groupe phényle.

2. Procédé de production d'imides d'acides maléiques substitués de formule (I)

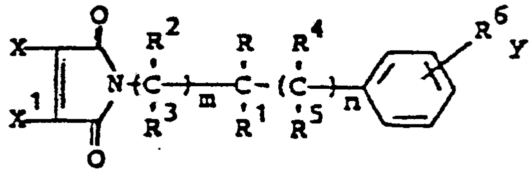

(I)

dans laquelle

X et $X^1$ représentent du chlore,

R est un groupe alkyle à chaîne droite ou ramifié ayant 1 à 4 atomes de carbone, tel que méthyle, éthyle, n-propyle, isopropyle, n-butyle, sec.-butyle, isobutyle et tertiobutyle, un groupe phenyle, benzyle ou phénéthyle portant éventuellement un à trois substituants égaux ou différents tels que méthyle, éthyle, chloro et bromo,

$R^1$ et $R^5$ sont identiques ou différents et représentent de l'hydrogène, un groupe alkyle à chaîne droite ou ramifié ayant 1 à 4 atomes de carbone, tel que méthyle, éthyle, n-propyle, isopropyle, n-butyle, isobutyle, sec.-butyle et tertio-butyle, un groupe phényle, benzyle ou phénéthyle portant éventuellement un à trois substituants égaux ou différents tels que méthyle, éthyle et chloro,

$R^6$ désigne du chlore, du brome, un groupe alkyle ayant 1 à 4 atomes de carbone tel que méthyle, éthyle, n-propyle, isopropyle, n-butyle, sec.-butyle, isobutyle et tertio-butyle, un groupe alkoxy ayant 1 à 3 atomes de carbone tel que méthoxy, éthoxy, n-propoxy et isopropoxy, un groupe sulfonylamino, alkylsulfonyle ou alkylcarbonyle ayant 1 à 3 atomes de carbone dans la partie alkyle, comme méthylsulfonyle, éthylsulfonyle, n-propylsulfonyle, isopropylsulfonyle, méthylcarbonyle, éthylcarbonyle, n-propylcarbonyle et isopropylcarbonyle, un groupe carbonylamino, trifluorométhyle, phényle, cyclopentyle ou cyclohexyle,

*m* et *n* ont la valeur 0, 1, 2 ou 3, la somme de *n* et *m* a la valeur 0, 1 ou 2 et *y* a la valeur 0, 1, 2 ou 3, à l'exception des composés lesquels X et $X^1$ représentent du chlore, *m* et *y* ont la valeur 0, *n* a la valeur 1, $R^1$, $R^4$ et $R^5$ représentent de l'hydrogène et R est un groupe méthyle, et des composés dans lesquels X, $X^1$ représentent du chlore, *m*, *n* et *y* ont la valeur 0 et $R^1$ représente de l'hydrogène et R est un groupe phényle, caractérisé en ce que:

a) on fait réagir un anhydride d'acide halogénomaléique de formule (II)

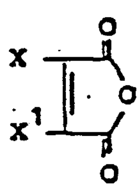

(II)

dans laquelle

X et $X^1$ ont la définition indiquée ci-dessus, avec des amines primaires de formule (III)

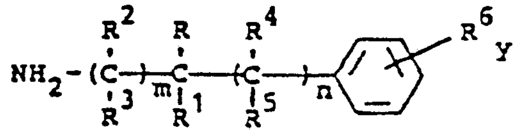

(III)

dans laquelle

R à $R^6$, *m, n* et *y* ont la définition indiquée ci-dessus, dans un diluant, ou bien
b) on fait réagir un ester dialkylique d'acide halogénomaléique de formule (IV)

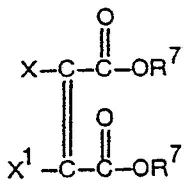

(IV)

dans laquelle
X et $X^1$ ont la définition indiquée ci-dessus, et
$R^7$ est un groupe alkyle, avec des amines primaires de formule (III), dans laquelle
R à $R^6$, *m, n* et *y* ont la définition indiquée ci-dessus, éventuellement dans un solvant ou un diluant, ou bien
c) on cyclise en les composés de formule (I) des mono-amides d'acides halogénomaléiques de formule (V)

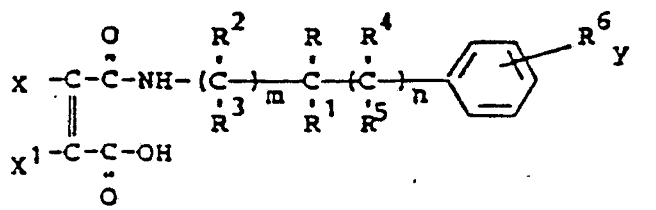

(V)

dans laquelle
X, $X^1$, R à $R^6$, *m, n* et *y* ont la définition indiquée ci-dessus,
en présence d'un solvant tel que, par exemple, l'acide acétique cristallisable et, le cas échéant, en présence d'un agent s'anhydrisant tel que, par exemple, l'acétanhydride ou le chlorure de thionyle.

3. Le N-[2-méthyl-3-(4-tertio-butylphényl)-propyl]-imide d'acide dibromomaléique.
4. Le N-3-phénylpentylimide d'acide dibromomaléique.
5. Le N-[1-méthyl-3-méthyl-3-(4-chlorophényl)-butyl]-imide d'acide dibromomaléique.
6. Compositions pesticides, caractérisées par une teneur en au moins un imide d'acide maléique substitué de formule (I) suivant les revendications 1 à 5.
7. Utilisation d'imides d'acides maléiques substitués de formule (I) suivant les revendications 1 à 5 pour combattre des parasites.
8. Procédé pour combattre des parasites, caractérisé en ce qu'on fait agir des imides d'acides maléiques substitués de formule (I) suivant les revendications 1 à 5 sur des parasites et/ou sur leur habitat.
9. Procédé de préparation de compositions pesticides, caractérisé en ce qu'on mélange des imides d'acides maléiques substitués de formule (I) suivant les revendications 1 à 5 avec des diluants et/ou des agents tensio-actifs.